Europäisches Patentamt

European Patent Office

Office européen des brevets

(11) Veröffentlichungsnummer: **0 267 497**
**A2**

(12) **EUROPÄISCHE PATENTANMELDUNG**

(21) Anmeldenummer: 87115888.7

(22) Anmeldetag: 29.10.87

(51) Int. Cl.4: **C07C 67/38** , C07C 69/533 , B01J 31/20

(30) Priorität: 08.11.86 DE 3638220

(43) Veröffentlichungstag der Anmeldung:
18.05.88 Patentblatt 88/20

(84) Benannte Vertragsstaaten:
**BE CH DE ES FR GB IT LI NL**

(71) Anmelder: **BASF Aktiengesellschaft
Carl-Bosch-Strasse 38
D-6700 Ludwigshafen(DE)**

(72) Erfinder: **Bertleff, Werner, Dr.
Neuzenlache 3
D-6806 Viernheim(DE)**
Erfinder: **Maerkl, Robert, Dr.
Schulstrasse 17
D-6701 Fussgoenheim(DE)**
Erfinder: **Kuehn, Gerhard
Am Weidenschlag 92
D-6700 Ludwigshafen(DE)**
Erfinder: **Panitz, Paul
Wachenheimer Strasse 1
D-6520 Worms 1(DE)**
Erfinder: **Kummer, Rudolf, Dr.
Kreuzstrasse 6
D-6710 Frankenthal(DE)**

(54) Verfahren zur Herstellung von Pentensäurealkylestern und deren Verwendung.

(57) Verfahren zur Herstellung von Pentensäurealkylestern durch Umsetzen von Butadien enthaltenden Kohlenwasserstoffgemischen mit Kohlenmonoxid und Alkanolen in Gegenwart von tertiären Stickstoffbasen und Kobaltcarbonylkatalysatoren bei einer Temperatur von 80 bis 150°C und unter einem Druck von 100 bis 1200 bar, dadurch gekennzeichnet, daß man 3-oder 4-Methylpyridin oder deren Gemische als Stickstoffbasen verwendet, und deren Verwendung.

## Verfahren zur Herstellung von Pentensäurealkylestern und deren Verwendung

Gegenstand der Erfindung ist ein Verfahren zur Herstellung von Pentensäurealkylestern durch Umsetzen von Butadien enthaltenden Kohlenwasserstoffgemischen mit Kohlenmonoxid und Alkanolen in Gegenwart von tertiären Stickstoffbasen und Kobaltcarbonylkatalysatoren bei einer Temperatur von 80 bis 150°C und unter einem Druck von 300 bis 1200 bar.

Entsprechend der DE-PS 26 30 086 erhält man Pentensäureester durch Umsetzen von Butadien enthaltenden $C_4$-Schnitten mit Alkanolen und Kohlenmonoxid in Gegenwart von tertiären Aminen mit einem $pK_a$-Wert von 3 bis 11 und Kobaltkatalysatoren. Die Carbalkoxylierung von Butadien zu Pentensäureestern und unter Mitverwendung von verschiedenen tertiären Aminen ist auch bereits aus Bulletin of the Chemical Society of Japan, Band 46, Seiten 524 bis 530 (1973) bekannt. Es hat sich jedoch herausgestellt, daß die Raumzeitausbeute bei der Reaktion verbesserungsbedürftig ist und es häufig zu Abscheidungen des Katalysators kommt.

Es war deshalb die technische Aufgabe gestellt, ein Verfahren zur Herstellung von Pentenäurealkylestern ausgehend von Butadien enthaltenden Kohlenwasserstoffgemischen zur Verfügung zu stellen, das mit hoher Raumzeitausbeute verläuft, Katalysatorabscheidungen vermeidet und einen hohen Umsatz an Butadien erzielt.

Diese technische Aufgabe wird gelöst in einem Verfahren zur Herstellung von Pentensäurealkylestern durch Umsetzen von Butadien enthaltenden Kohlenwasserstoffgemischen mit Kohlenmonoxid und Alkanolen in Gegenwart von tertiären Stickstoffbasen und Kobaltcarbonylkatalysatoren bei einer Temperatur von 80 bis 150°C und unter einem Druck von 300 bis 1200 bar, bei dem man 3-oder 4-Methylpyridin oder deren Gemische als tertiäre Stickstoffbasen verwendet.

Das neue Verfahren hat den Vorteil, daß man hohe Raumzeitausbeuten erzielt. Weiter hat das neue Verfahren den Vorteil, daß Cobaltsalzabscheidungen während der Reaktion und bei der Aufarbeitung des Reaktionsgemisches vermieden werden, ferner hat das neue Verfahren den Vorteil, daß man hohe Umsätze an Butadien erzielt.

Bevorzugte Butadien enthaltende Kohlenwasserstoffgemische sind $C_4$-Schnitte. Dies sind Gemische von überwiegend unverzweigten $C_4$-Kohlenwasserstoffen mit mehr als 10 Gew.-% Butadien und mehr als 15 Gew.-% Butenen. Je nach Provenienz liegen die einzelnen Komponenten in solchen Gemischen normalerweise in folgenden Anteilen vor.

Butadien 10 bis 70, durchschnittlich 40 bis 60 Gew.-%.

Isobuten 15 bis 40, durchschnittlich 20 bis 35 Gew.-%

But-1-en 10 bis 40, durchschnittlich 10 bis 25 Gew.-%

But-2-en 5 bis 20, durchschnittlich 5 bis 15 Gew.-%

Butane 1 bis 10, durchschnittlich 1 bis 10 Gew.-%

Butine 0,1 bis 3, durchschnittlich 0,1 bis 3 Gew.-%

Solche $C_4$-Schnitte fallen beispielsweise an bei der Dehydrierung von Butan oder Buten oder als Nebenprodukte bei der thermischen Spaltung von Leichtbenzin oder höheren Kohlenwasserstoffschnitten.

Geeignete Alkanole haben in der Regel 1 bis 6 Kohlenstoffatome, insbesondere 1 bis 4 Kohlenstofatome wie Methanol, Ethanol, Propanol, Butanol oder Isobutanol. Besonders bevorzugt wird Methanol verwendet. Vorteilhaft verwendet man einen Überschuß an Alkanolen an, insbesondere 1,1 bis 5 Mol je Mol Butadien.

Die Umsetzung führt man bei einer Temperatur von 80 bis 150°C, insbesondere 110 bis 145°C durch. Ferner hält man einen Druck von 100 bis 1200, vorteilhaft von 300 bis 1200 bar, insbesondere 300 bis 900 bar ein.

Kohlenmonoxid wird vorteilhaft im Überschuß, z.B. dem 1,5 bis 10-fachen der stöchiometrisch erforderlichen Menge angewandt.

Die Umsetzung wird in Gegenwart von Kobaltcarbonylkatalysatoren durchgeführt. Diese werden entweder in situ aus Kobaltsalzen, z.B. fettsauren Kobaltsalzen wie Formiat, Acetat, Propionat oder Butyrat erzeugt. Vorteilhaft bringt man den Katalysator bereits als Kobaltcarbonyl ein. Insbesondere hat es sich bewährt, wenn man Kobaltcarbonylkatalysator gelöst in dem Butadien enthaltenden Kohlenwasserstoffgemisch in das Reaktionsgemisch einbringt. Eine solche Lösung erhält man beispielsweise durch Umsetzen einer wäßrigen Lösung von fettsaure Kobaltsalzen mit einem Gemisch aus Kohlenmonoxid und Wasserstoff in Gegenwart von Aktivkohle bei einer Temperatur von 100 bis 200°C und einem Druck von 100 bis 400 bar. Aus der wäßrigen Lösung wird der entstandene Kobaltcarbonylkatalysator dann mit Butadien enthaltendem Kohlenwasserstoffgemisch extrahiert.

Es hat sich besonders bewährt, wenn man ein Molverhältnis von Butadien zu Kobaltkatalysator von 1 : 0,01 bis 1 : 0,3, vorteilhaft von 1 : 0,01 bis 1 : 0,1, insbesondere 1 : 0,04 bis 1 : 0,08 einhält.

Die Umsetzung wird in Gegenwart von 3-oder 4-Methylpyridin oder deren Gemischen durchgeführt. Besonders bevorzugt ist 3-Methylpyridin. Vorteilhaft wendet man je Mol Cobaltcarbonylkatalysator 0,7 bis 2 Mol der genannten Methylpyridine an.

Bei der Umsetzung erhält man vorwiegend Pent-3-ensäurealkylester und untergeordnete Mengen an Pent-4-ensäurealkylester und Pent-2-ensäurealkylester. Demzufolge sind Pent-3-ensäurealkylester bevorzugte Verfahrenserzeugnisse.

Vorteilhaft verwendet man das so erhaltene Pentensäurealkylester enthaltende Reaktionsgemisch nach Abtrennen überschüssiger Kohlenwasserstoffe und gegebenenfalls des größten Teils an 3-oder 4-Methypyridin, ohne oder partieller Abtrennung des Kobaltkatalysators zur Herstellung von Adipinsäurealkylestern durch weitere Umsetzung mit Alkanolen und Kohlenmonoxid sowie den verbleibenden Mengen an 3-oder 4-Methylpyridin oder deren Gemische.

Das Verfahren nach der Erfindung sei an folgenden Beispielen veranschaulicht.

Vergleichsbeispiele 1 und 2 und Beispiele 1 bis 4

Die Umsetzung wurde in 2 hintereinander geschalteten Schlaufenreaktoren mit jeweils 68,5 l Inhalt durchgeführt. C₄-Schnitt mit einem Gehalt von 41,5 Gew.-% Butadien, 3-Methylpyridin bzw. Pyridin, Methanol sowie Kobaltcarbonyl wurden vorgemischt zusamment mit der erforderlichen Menge Kohlenmonoxid kontinuierlich dem ersten Reaktor zugeführt. In beiden Reaktoren wird eine Temperatur von 135°C und ein Druck von 650 bar eingehalten. Der dem zweiten Schlaufenraktor entnommene Reaktionsaustrag wurde dreistufig entspannt und durch Destillation aufgearbeitet. Die Zufuhr an C₄-Schnitt wurde so eingestellt, daß Butadien jeweils zu 99 % umgesetzt wurde. Der Austrag wurde auf Ausscheidungen untersucht. Die näheren Einzelheiten und Ergebnisse sind aus folgender Tabelle ersichtlich. Das Molverhältnis von Butadien, Methanol, Kohlenmonoxid und Stickstoffbase betrug jeweils 1 : 1,1 : 3 : 1. Der eingesetzte Kobaltkatalysator bestand zu 50 % aus rückgeführtem Sumpfprodukt der Katalysatorabtrennung und zu 50 % aus frischem Kobaltcarbonyl, das durch Reduktion von Kobaltacetat mit Kohlenmonoxid und Wasserstoff und anschließende Extraktion mit dem C₄-Schnitt erhalten wurde.

Tabelle

| | N-Base | Butadien | Butadien: Kobalt Mol/Mol | Kobalt-gehalt (% mm) | Niederschlag | RZA kg PSE/lRR x h |
|---|---|---|---|---|---|---|
| | | (kg/h) | | | | |
| Vergleichs-1 beispiel | Pyridin | 5,2 | 1 : 0,057 | 1,2 | nein | 0,073 |
| Vergleichs-2 beispiel | Pyridin | 5,2 | 1 : 0,071 | 1,5 | ja | - |
| Beispiel 1 | 3-Methyl-pyridin | 5,3 | 1 : 0,055 | 1,2 | nein | 0,074 |
| Beispiel 2 | 3-Methyl-pyridin | 5,3 | 1 : 0.070 | 1,53 | nein | 0,074 |
| Beispiel 3 | 3-Methyl-pyridin | 5,3 | 1 : 0,076 | 1,66 | nein | 0,074 |
| Beispiel 4 | 3-Methyl-pyridin | 6,2 | 1 : 0,079 | 1,72 | nein | 0,087 |

RZA = Raumzeitausbeute

PSE = Pentensäuremethylester (alle Isomere)

(1) = der Kobaltgehalt wurde nach Entspannung auf Atmosphärendruck und Abtrennung von restlichen Kohlenwasserstoffen bestimmt.

Fazit: Während mit Pyridin als Katalysator Cobaltkonzentrationen von ca. 0,9 % im Reaktionsgemisch bzw. 1,2 % im vom Rest-$C_4$-Schnitt befreiten Austrag nicht überschritten werden können, gelingt mit 3-Methylpyridin eine wesentliche Steigerung der Cobaltlöslichkeit.

## Ansprüche

1. Verfahren zur Herstellung von Pentensäurealkylestern durch Umsetzen von Butadien enthaltenden Kohlenwasserstoffgemischen mit Kohlenmonoxid und Alkanolen in Gegenwart von tertiären Stickstoffbasen und Kobaltcarbonylkatalysatoren bei einer Temperatur von 80 bis 150°C und unter einem Druck von 100 bis 1200 bar, dadurch gekennzeichnet, daß man 3-oder 4-Methylpyridin oder deren Gemische als Stickstoffbasen verwendet.

2. Verfahren nach Anspruch 1, dadurch gekennzeichnet, daß man je Mol Cobaltcarbonylkatalysator 0,7 bis 2 Mol 3 oder 4-Methylpyridin oder deren Gemische einsetzt.

3. Verfahren nach den Ansprüchen 1 und 2, dadurch gekennzeichnet, daß man 3-Methylpyridin verwendet.

4. Verfahren nach den Ansprüchen 1 bis 3, dadurch gekennzeichnet, daß man eine Temperatur von 110 bis 145°C einhält.

5. Verfahren nach den Ansprüchen 1 bis 4, dadurch gekennzeichnet, daß man einen Druck von 450 bis 900 bar einhält.

6. Verwendung von Pentensäurealkylester enthaltenden Reaktionsgemischen, die nach dem Verfahren nach den Ansprüchen 1 bis 5 erhalten worden sind, nach Abtrennung der überschüssigen Kohlenwasserstoffe ohne oder partieller Abtrennung der Kobaltkatalysatoren zur Herstellung von Adipinsäurealkylestern durch Umsetzen mit Kohlenmonoxid und Alkanolen.